(19) **Europäisches Patentamt**

**European Patent Office**

**Office européen des brevets**

(11) **EP 1 176 993 B1**

(12) **EUROPEAN PATENT SPECIFICATION**

(45) Date of publication and mention
of the grant of the patent:
**04.06.2003 Bulletin 2003/23**

(51) Int Cl.7: **A61L 27/18**, A61L 27/56

(21) Application number: **00925547.2**

(86) International application number:
**PCT/IE00/00056**

(22) Date of filing: **08.05.2000**

(87) International publication number:
**WO 00/067813 (16.11.2000 Gazette 2000/46)**

(54) **BIOSTABLE POLYURETHANE PRODUCTS**

BIOSTABILE POLYURETHANPRODUKTE

ARTICLES EN POLYURETHANNE STABLES DU POINT DE VUE BIOLOGIQUE

(84) Designated Contracting States:
**AT BE CH CY DE DK ES FI FR GB GR IE IT LI LU MC NL PT SE**

(30) Priority: **07.05.1999 WOPCT/IE99/00037**
**07.05.1999 WOPCT/IE99/00038**

(43) Date of publication of application:
**06.02.2002 Bulletin 2002/06**

(73) Proprietor: **Salviac Limited**
**Dublin 2 (IE)**

(72) Inventors:
• **BRADY, Eamon**
**Elphin, County Roscommon (IE)**
• **CANNON, Ann, Marie**
**Pettigo, County Donegal (IE)**
• **FARRELL, Fergal**
**Athy, County Kildare (IE)**

(74) Representative: **O'Brien, John Augustine et al**
**John A. O'Brien & Associates,**
**Third Floor,**
**Duncairn House,**
**14 Carysfort Avenue**
**Blackrock, Co Dublin (IE)**

(56) References cited:
**EP-A- 0 297 252**     **EP-A- 0 778 029**
**WO-A-98/20939**     **US-A- 4 600 652**

**Description**

This invention relates to biostable biocompatible polyurethane implants suitable for short and long term implantation in vivo.

Introduction

Known medical grade polyether polyurethane elastomers are susceptible to phagocyte mediated Environmental Stress Cracking (ESC). Because of this known surface phenomenon such polyether polyurethanes are not suitable candidates to provide a foam implant. Conventional polyurethane foam structures have ether linkages which are subject to oxidation induced by an inflammatory response in vivo. This is the first stage in ESC which for foam materials ultimately represents a complete loss of material and device integrity.

There is therefore a need for a polyurethane based foam implant material which will overcome these problems.

In particular it is an object of the current invention to provide a new class of low density biostable polymers suitable for implantation.

Statements of Invention

According to the invention there is provided a biostable porous polyurethane foam implant material, the material containing isocyanurate linkages derived from diphenyl methane diisocyanate (MDI) and having a cross-linked structure, and a void content of at least 85%, preferably in excess of 90%, most preferably in excess of 95%, and ideally in excess of 97%.

In particular the invention provides a biostable porous polyether or polycarbonate polyurethane article characterised in that the polyurethane is manufactured from diphenyl methane diisocyanate (MDI) with a 2, 4 MDI isomer content of less than 3%, difunctional polytetramethylene ether glycol (PTMEG) or a polycarbonate polyol, a chain extender, water, a cross-linking agent, and a trimerisation agent, a blowing and/or gelling catalyst and a surfactant. The porous biomaterial is characterised in that it has a void content in excess of 85%, is cross-linked, the hard phase contains isocyanurate linkages and the materials are biostable.

We have surprisingly been able to produce low density polyether and polycarbonate polyurethane porous foam that is highly biostable. The biostability of the materials of the invention is especially surprising in view of the known problems with conventional polyether polyurethanes and the high surface area to volume ratio of the materials. The excellent biostability of the soft phase of the material is attributable, in part, to the method of manufacture. The material is prepared in a manner whereby the molecules in the material are virtually stress free. The stress free configuration of the molecules of the system provides the highest activation energy barrier to degradation. The materials are processed using a reaction blow moulding free rise method. The stability of the hard phase is ensured by the incorporation of highly stable isocyanurate linkages. A high level of cross-linking in the hard phase enhances its stability.

The cross-linking agent is employed at high concentrations so as to stabilise the foam. These cross linking agents are highly reactive due to the presence of an amine linkage in their structure. They react with isocyanate at an early stage in the curing process. This helps to capture the blow and prevent blow-off.

The trimerisation catalyst causes three isocyanates to trimerise into an isocyanurate structure. This adds additional functionality and is particularly important because the reaction is temperature dependent. This reaction is most aggressive in the region of maximum exotherm. This corresponds to the time in the reaction when the blow is at a maximum. This additionally helps to stabilise the foam at the most critical point in the process and together with the cross-linking agent leads to rapid structure building at this time.

The high isocyanate index ensures that the isocyanate is in excess and this facilitates the trimerisation reaction and the triethanolamine reaction. The excess isocyanate provides the final cross-linking during the post cure phase of the process. At this stage in the reaction allophanate and/or biuret linkages are formed.

In a preferred embodiment the cross-linking agent is an alkanol amine. More preferably the cross-linking agent is triethanolamine.

In a preferred embodiment the triethanolamine content is between 1% and 5% of the formulation mass. More preferably the triethanolamine content is between 1% and 3% of the mass of the formulation.

In a preferred embodiment a trimerisation catalyst is employed. The trimerisation catalyst may be a tertiary amine, an organophosphorous compound, a metal alkyl or a carboxylate. Carboxylates are preferred and potassium acetate is the most preferred trimerisation catalyst.

In a preferred embodiment the amount of potassium acetate is between 0.02% and 0.12% of the mass of the formulation. More preferably the potassium acetate content is between 0.06% and 0.07% of the mass of the formulation.

The invention also provides a process for preparing an implant of the invention, the processing comprising;

preparing an isocyanate terminated prepolymer in an excess of MDI with a 4, 4 MDI isomer content of greater than 97%;

preparing a diol reaction mixture comprising a diol, a cross linking agent, a trimerising catalyst, water, a blowing and/or gelling catalyst, and a surfactant;

mixing the prepolymer and the diol;

dispensing the mixed reaction ingredients into a preheated mould which permits free rise of the foam;

post curing the foam;

demoulding the foam; and

machining the foam to form an implant.

Various other features of the invention are given in the claims.

Brief Description of the Drawings

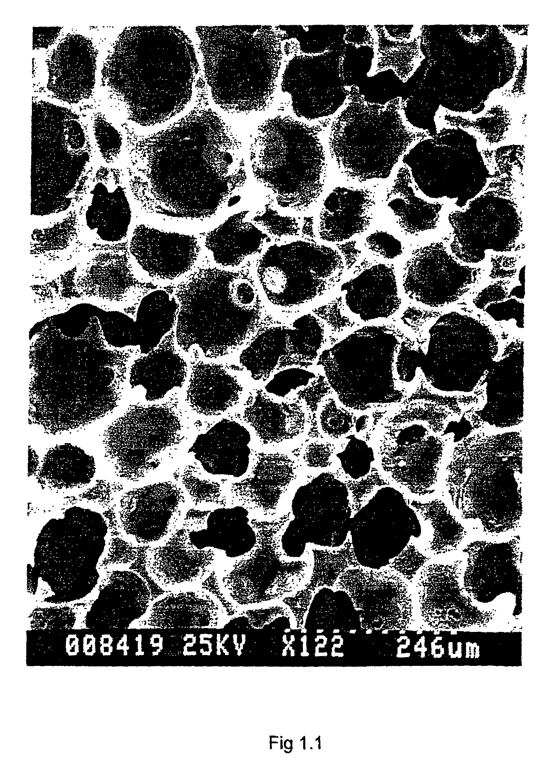

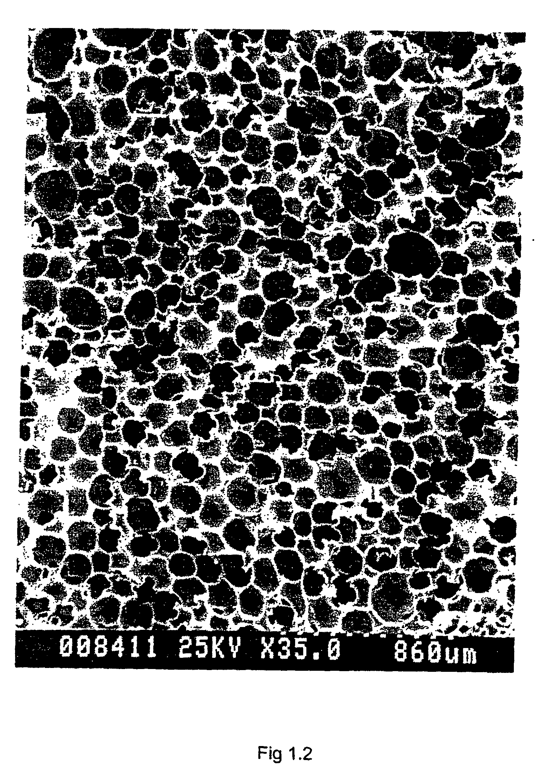

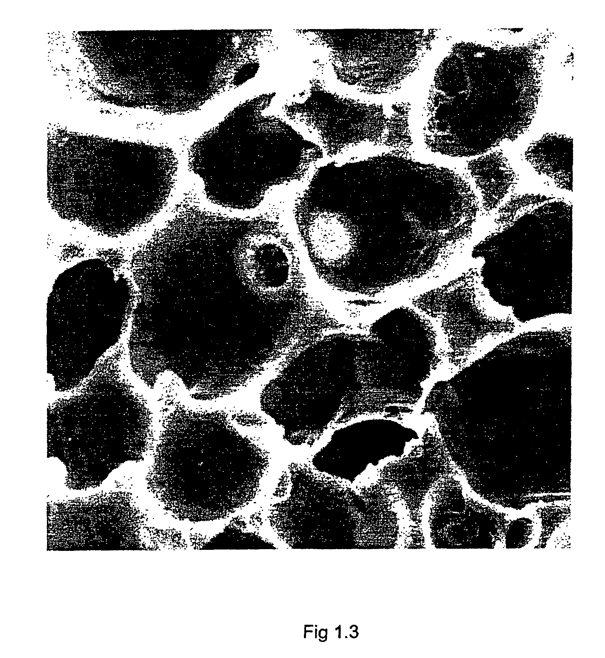

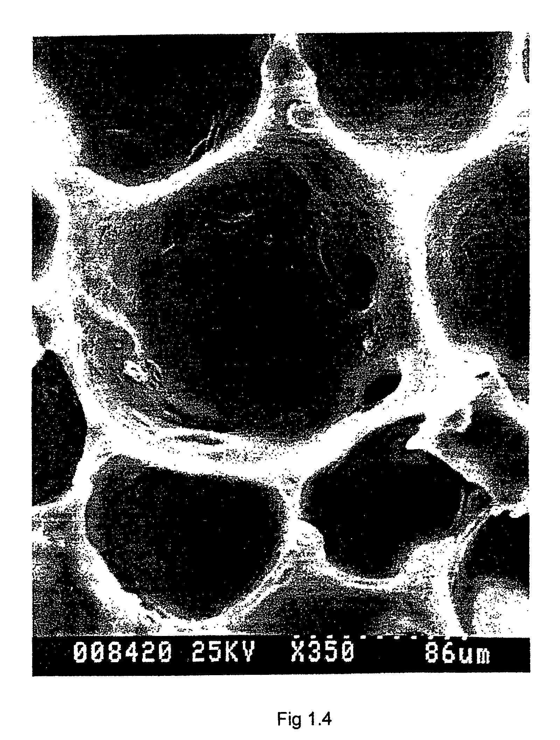

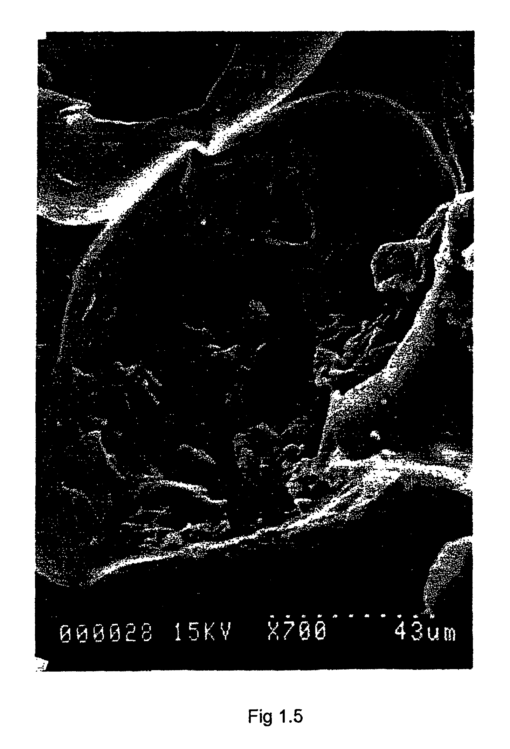

Fig. 1.1 is a SEM image of Polyether polyurethane biomaterial placed in a chamber with a 0.45μm filter, implanted subcutaneously in the rat animal model and explanted after 8 weeks. The integrity of the voids of the biomaterial will be noted. The structure of the voids is similar to that for the control sample (Fig. 1.2) demonstrating no evidence of ESC;

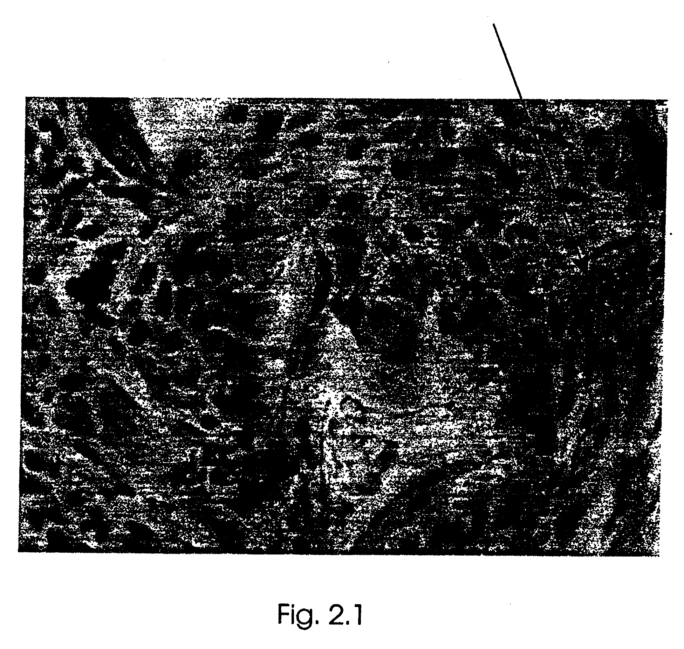

Fig. 1,2 is a SEM image of Polyether polyurethane biomaterial control sample which was not implanted in the rat model;

Fig. 1.3 is a SEM image of Polyether polyurethane biomaterial placed in a chamber with a 0.45μm filter, implanted subcutaneously in the rat animal model and explanted after 8 weeks. As in Fig. 1.1, the integrity of the voids of the biomaterial will be noted. The structure of the voids is similar to that for the control sample (Fig. 1.2) demonstrating no evidence of ESC;

Fig. 1.4 is a SEM image (at high magnification) of Polyether polyurethane biomaterial placed in a chamber with a 0.45μm filter, implanted subcutaneously in the rat animal model and explanted after 8 weeks. The integrity of the void of the biomaterial will be apparent. The structure of the voids is similar to that for the control sample (Fig. 1.2) demonstrating no evidence of ESC;

Fig. 1.5 is a SEM image of Polyether polyurethane biomaterial placed in a chamber with a 3.0μm filter, implanted subcutaneously in the rat animal model and explanted after 26 weeks. The integrity of the voids of the biomaterial and the cellular deposition on the biomaterial will be noted. There is no evidence of ESC;

Fig. 2.1 is a photomicrograph of the scaffold of Example 1 stained with Haemoxylin and Eosin (H&E), 12 weeks following implantation. The presence of numerous macrophage cells throughout the scaffold and the presence of blood capillaries (indicated by the arrow) in the centre of the scaffold will be noted;

Detailed Description

The invention provides a biostable porous polyether or polycarbonate polyurethane article characterised in that the polyurethane is manufactured from diphenyl methane dissocyanate (MDI) with a 2, 4 MDI isomer content of less than 3%, difunctional polytetramethylene ether glycol (PTMEG) or a polycarbonate polyol, a chain extender, water a cross-linking agent and a trimerisation agent. The porous biomaterials are characterised in that it has a void content in excess of 80%, is cross-linked, the hard phase contains isocyanurate linkages and the materials are biostable.

The biomaterials of this invention have unique physical and chemical characteristics that make them very suitable for in vivo implantation. The materials are especially suitable to transcatheter implantation due to the extremely high void content. Thus, the implants may be used, for example, as an occuluder or a tissue bridge.

MDI is the preferred isocyanate of this invention. The condensation of aniline with formaldehyde may produce MDI containing 2,4 and 4'4 isomers. MDI is offered in a wide variety of products, which include the 4,4 isomer 'pure' MDI product, and a variety of polymeric MDI for various different applications

Crude (polymeric) MDI is the product formed from the initial production stages where no purification has been carried out to separate various isomers and higher polymeric forms. As an example Emerck crude MDI contains the following components: 55% 4,4 and 2,4 diphenylmethane diisocyanate, 25% triisocyanate, 20% polymeric isocyanate. Due to the myriad of structures within the crude material it is preferable to use a pure grade of MDI for materials of this invention.

Pure MDI is classified as an isomer mixture of 4,4' diphenylmethane diisocyanate and 2,4' diphenylmethane diisocyanate. For the materials of this invention the 4,4' isomer is preferred. The pure MDI can be obtained as a solid, a solid in flake form or as a liquid. Pure 4,4'-MDI is sensitive to heat and will dimerise. For pure solid MDI storage below 5°C is recommended and although this will slow down the rate of dimerisation, there is still a limited time for which the pure MDI can be stored. Below 5°C a shelf life of approximately 6 months is recommended.

For the biomaterials of this invention it is preferred that pure Flake MDI is used with an NCO content between 33.4% to 33.7%, a 2'4 MDI content <3%, a dimer content of <1%, a purity of 99.5% and a hydrolysable chlorine content of <50ppm. In an embodiment of this invention the isocyanate in the reaction chamber is in excess. More preferably the isocyanate index is between 1.03 and 1.20. Even more preferably the isocyanate index is between 1.06 and 1.16. Most preferably the isocyanate index is 1.13.

The most important characteristics of a polyol for the production of a low density foam are:

- The hydroxyl group (or amine) should be reactive to isocyanate.
- The viscosity should be as low as possible relative to its molecular weight and functionality.
- Preferably the polyol should have a functionality in the region of 3.

The reactivity of the hydroxyl group is dependent on whither it is a primary, tertiary, or quaternary -OH group. For example the reactivity of secondary hydroxyl end groups is several times less reactive with isocyanate than primary hydroxyl groups.

The viscosity of the polyol needs to be as low as possible. The table below shows the viscosities of a number of commercially available polyols. The viscosity clearly depends on molecular weight and functionality. The importance of viscosity lies in the fact that the resistance to blowing is determined by the viscosity. Polyols with reduced viscosity are preferred for low density applications.

| Material | Supplier | Molecular Weight | Viscosity |
|---|---|---|---|
| PPG Diol | Lyondell | 1000 | 110 to 220cps @ 25'C |
| PPG Diol | Lyondell | 2245 | 350 to 700 cps @ 25'C |
| PPG Triol | Lyondell | 1070 | 400cps @ 25'C |
| PTMEG Diol | Du Pont | 1000 | 250 to 320cps @40'C |
| PTMEG | Du Pont | 2000 | 950-1450 cps @ 40'C |
| CX5510 (Polycarbonate diol) | Nissei Chemicals | 1000 | 1610cps @50'C |
| CX5520 (Polycarbonate Diol) | Nissei Chemicals | 2000 | 10,400 cps @50'C |

The functionality of the polyol is crucial to stabilising the expanded formulation. High functionality increases the rate at which molecular weight is developed. This molecular weight increase prevents the internal pressure of the gas from blowing the foam apart. This molecular weight increase also prevents the foam from shrinking as the blowing gas is liberated during the cell opening phase.

The invention provides a range of biostable materials based on these polyols. In particular low density foams using 'tertiary carbon' free polyols are provided.

Multifunctional polyols are usually prepared using a trifunctional initiator, such as glycerol or trimethylolpropane. These materials contain tertiary carbons and would be susceptible to attack and degradation by phagocytic agents. The absence of these linkages enhances the biostability of the materials. Difunctional polyols are thus advantageous from an implantation biostability stand point. This is especially important where the implant is of high surface area and low density.

The lack of functionality and the high relative viscosity of polytetramethylene ether diols and polycarbonate diols make the blowing and stabilisation of low density foams from these materials extremely difficult. The complexity is added to by the need of the resulting foams to have excellent biostability characteristics.

High density PTMEG foams have been produced for applications in shoe manufacture. Du Pont have produced PTMEG microfoams with a density range from $250kg/m^3$ to $300kg/m^3$. Low density foam is defined per this invention as having a void content in excess of 80%. Soft microcellular polycarbonate urethane foams are also described by Pinchuck EP461 375.

The invention provides a range of low density PTMEG and polycarbonate polyol based polyurethanes with properties suitable for long term biomedical implantation.

The PTMEG materials of this invention capture the excellent resilience properties of PTMEG and overcome the biostability issues of conventional polyether urethanes.

PTMEG is the preferred polyether polyol of this invention. PTMEG is manufactured from Polytetrahydrofuran (PTHF) by a ring opening polymerisation technique. The linearity of PTMEG increases the difficulty in preparing low density 3 dimensional porous matrices with this material. The linearity results from the fact that no polyfunctional initiator is used during the polymerisation step. This is unlike the polymerisation of other polyethers used in polyurethane foam manufacture that can use a trifunctional or higher functionality initiator to increase the functionality of the polyether polyol.

The PTMEG polyols used in this invention have molecular weights of 400 - 5000, more preferably between 500-2000.

PTMEG is a white waxy solid material at low temperatures and has a melt temperature ranging from 11°C to 43°C depending on the molecular weight of the PTMEG.

The quality of PTMEG starting materials is important for the biomaterials of this invention. The preferred molecular weights of the polyols for this application are 650, 1000 and 2000 with minimum polydispersity. Ideally the PTMEG starting materials will have characteristics as outlined below;

| Characteristic | 650 molecular weight PTMEG | 1000 molecular weight PTMEG | 2000 molecular weight PTMEG |
|---|---|---|---|
| Molecular weight | 600-700 | 950-1050 | 1900-2100 |
| OH value | 160-187 | 112-118 | 53 -59 |
| Water content | <0.015 % wt | <0.015 % wt | <0.015 % wt |
| Viscosity @40°C cP | 100 - 200 | 260 - 320 | 950 - 1450 |
| Antioxidants | <0.1% | <0.1% | <0.1% |

The development of molecular weight during the foaming step is a critical aspect of the invention. The importance of this area results from the following limitations of PTMEG as a foaming material:

• PTMEG has lower reactivity than standard foaming polyols.
• PTMEG is commercially available with a functionality of 2. Standard PPG foaming polyols have functionalities above 3 and as high as 8.
• PTMEG is significantly more viscous than standard PPG based foaming polyols

The preparation of PTMEG and polycarbonate foams with void contents in excess of 90%, 95% and 97% is also unexpected. This has been achieved to spite the limitations of difunctional, high viscosity polyols as a foaming ingredient. A strong blowing catalyst is used to deal with the high relative viscosity of the polyol. This pushes the blowing reaction in the early stages of foaming, while the molecular weight is low. Normally this approach would exacerbate the lack of functionality of the polyol and the foam would be blown apart towards the end of the blowing phase. However we have introduced mechanisms for developing the molecular weight to deal with the fast blowing reaction. These mechanisms include the use of a reactive cross-linking agent and the incorporation of a trimerisation catalyst. The cross-linking agent provides functionality early in the blowing process. The inclusion of a trimerisation catalyst ensures cyclic isocyanurates groups form during the later part of the blowing phase. The incorporation of isocyanurates introduces a further element of cross-linking and provides additional stability to the foam. The trimerisation catalyst lowers the temperature for the formation of isocyanurates. The rate of isocyanurate formation increases with temperature and is thus linked to the exotherm, the material and the mould temperatures.

The polycarbonate diols of the invention have the following general characteristics:

• The polycarbonate diols are difunctional polyols.
• They are free of tertiary carbons.
• They have molecular weights of from 400 to 5000.
• They are linear unbranched polycarbonate diols.

The diols of the invention are generally manufactured by a reaction of a carbonate with a diol or a mixture of diols. Examples of the polycarbonate polyols used in the invention are disclosed by Greco EP 0 533275 , Lia U.S. 4,131,731 and Funakoshi EP 0 754 714 which are included herein in their entirety for reference. Preferred diols are linear aliphatic diols. Preferably the polycarbonate has between 4 and 20 carbons between carbonate linkages. More preferably between 4 and 10.

The materials and processes of the invention allow for the early expansion of the foam in a low viscosity state. As the formulation reaches full blow molecular weight is developed quickly and this stabilises the expanded material. The rapid development of molecular weight at the time of full blow is achieved by building three separate types of functionality into the formulation.

1. The incorporation of a reactive cross-linking agent into the formulation provides tri-functionality early in the process.

2. A trimerisation catalyst generates trifunctional isocyanurates at the time of maximum blow. This occurs because of the temperature dependent nature of the trimerisation reaction and it is accelerated by the exotherm generated in the blowing reaction. The materials and mould temperatures are important in optimising this portion of the reaction.

3. The excess isocyanate reacts with urethane linkages already formed. This provides cross-links in the later stages of the process.

It is the combination of these three events that makes the stabilisation of the foams of the invention possible.

The cross-linking agent is a reactive component that has a functionality of three. It forms covalent bonds when reacted with the diisocyanate, resulting in the formation of a three dimensional network within the material.

Cross-linking agents can be grouped into three classes, alcohols, amines and alkanol amines.

The alkahols include glycerol, trimethylol propane, and sorbitol. As all of these contain tertiary carbons they are not preferred for the manufacture of the biostable foams of the invention.

The amines include MBOCA (3,3'-dichloro-4,4'-diamino-diphenylmethane) and diethylenetriamine.

Alkanol amines include ethanol amine, diethanolamine and triethanolamine.

Alkanol amines are preferred per this invention. A characteristic of these cross-linking agents is that they function as catalysts, thus they maybe classified as reactive catalysts. A high purity grade is preferred (99+%).

Water is employed as the blowing agent in the materials of the invention. The quality of the water used in this formulation is important and is controlled. HPLC grade water with a residue on evaporation of < 0.0005% may be used. However, more preferably deionised water with no detectable oxidisable substances, chlorides, nitrates sulphates and ammonium, < 0.001% of residue on evaporation, pH 5 - 8 and endotoxin levels < 0.5EU/ml is used. High pyrogen levels in starting materials of products destined for human implantation should be avoided. The water content of the polyol resin component ranges from 2-5 php (parts per hundred polyol) depending upon the void content of biomaterial required.

There are two main classes of catalysts in the manufacture of polyether polyurethanes.

♦ Organic tin catalysts - primarily accelerate the gelling reaction
♦ Tertiary amine compounds - to promote both the gelling and the blowing reactions

Amine catalysts are preferred for this invention. Depending on the structure of the amine either the gelling or blowing reaction is favoured. By careful selection of the catalysts a desirable rate between the gelling and blowing reactions can be achieved to yield a low density material.

Amine catalysts including but not limited to triethylenediamine, N,N,N'N'-tetramethylhexamethylenediamine, bis(2-dimethylaminoethyl)methyl amine, N-(2-dimethylaminoethyl)-N'-methylpiperazineand N-(2-dimethylaminoethyl) ether can be used.

The preferred catalyst system for this invention is Desmorapid PP (From Whitchem), which is a combination of bis(2-dimethylaminoethyl)methyl amine and N-(2-dimethylaminoethyl)-N'-methylpiperazine, with triethylenediamine. The resulting combination yields a strong blowing reaction.

A further class of catalysts is based on carboxylates. These promote the trimerisation reaction of the isocyanate and are critical to the formation of the stable foams of the invention. The inclusion of a trimerisation catalyst ensures cyclic isocyanurates groups form during the later part of the blowing phase. The incorporation of isocyanurates introduces a further element of cross-linking and provides additional stability to the foam. The trimerisation catalyst lowers the temperature for the formation of isocyanurates. The rate of isocyanurate formation increases with temperature and is thus linked to the exotherm, the material and the mould temperatures.

The trimerisation catalyst causes three isocyanates to trimerise into an isocyanurate structure. This adds additional functionality and is particularly important because the reaction is temperature dependent. This reaction rate is fastest in the region of maximum exotherm. This corresponds to the time in the reaction when the blow is at a maximum. This additionally helps to stabilise the foam at the most critical point in the process and together with the cross-linking agent leads to rapid structure building at this time.

The high isocyanate index ensures that the isocyanate is in excess and this provides raw material for the trimerisation reaction and the cross linking reaction.

In a preferred embodiment a trimerisation catalyst is employed. The trimerisation catalyst may be a tertiary amine, an organophosphorous compound, a metal alkyl or a carboxylate. Carboxylates are preferred and potassium acetate is the most preferred trimerisation catalyst.

In a preferred embodiment the amount of potassium acetate is between 0.02% and 0.12% of the mass of the formulation. More preferably the potassium acetate content is between 0.06% and 0.07% of the mass of the formulation.

Catalysts such as potassium acetate, sodium acetate and the like are added to the resin in a solvent carrier.

The function of the surfactant is to control the formation and growth of the gas bubbles and with it many processing and final properties of the scaffold.

In the emulsification of the raw materials, surfactants allow thermodynamically incompatible components of a polyurethane foam formulation to mix. The concentration of gas bubbles formed during the foaming is strongly dependent on the characteristics of the surfactant used.

During the rise of the foam the main demand on the surfactant is the stabilisation of the expanding liquid mixture. The surfactant reduces the surface tension of the liquid mixture and therefore reduces the energy the system needs to facilitate cell formation and growth.

During the rise the viscosity increases gradually within the material. The structural stability increases with this viscosity increase and the structure becomes more self-supporting. As the blow reaches its maximum the spherical voids start to impinge on one another. The surfaces of infringement become locally flattened. The surfactant plays a critical role at this stage. By controlling the surface tension some of the flattened surfaces of the impinging voids rupture. As these rupture the material in the voids reflows into the main arch.

A typical silicone surfactant generally has a siloxane backbone formed by dimethylsiloxane units. Polyether groups and/or additional modifications can be attached to the siloxane backbone. Suitable surfactants include BF 2270, BF 8002 from Goldschmidt A.G. Surfactant quantities from 0.30% to about 2% weight percent based on polyol resin may be suitable. More preferably the weight % of the surfactant is between 0.5% and 1.2%.

Chain extenders are low molecular weight difunctional compounds. During the reaction hard segments are formed by the reaction of the diisocyanate with the low molecular weight chain extender. Water used as a blowing agent can also be considered a chain extender as it is a low molecular weight difunctional component. Diols are the preferred chain extender.

1-4-butane diol is a preferred chain extender. A high purity grade is required (99+%), as impurities such as 2-methyl butane-1-4-diol should be avoided.

Other diol chain extenders that can be considered are, but not limited to, ethylene glycol, propylene glycol, 1-6-hexane diol, diethylene glycol, dipropylene glycol.

The materials of the invention are manufactured by a quasi-prepolymer method. In the production of an isocyanate quasi prepolymer the diisocyanate is reacted with a portion of polyol to form a relatively low viscosity prepolymer which is then subsequently reacted with the remainder of the polyol, water, various catalysts, surfactants and chain extenders and other ingredients.

The key outputs from the preparation of the prepolymer process are the NCO content of the prepolymer and the resulting viscosity of the prepolymer. These outputs are controlled in the process by controlling the temperature and the method of preparation. The prepolymer is preferably prepared at a temperature between 70°C and 80°C. This provides the best control of viscosity and minimises side reactions. Possible undesirable side reactions at this stage include the formation of allophanates and trimerisation.

The method in which the prepolymer is prepared has an affect on the viscosity of the prepolymer. If the liquid polyol is added to isocyanate a lower viscosity prepolymer results than if the isocyanate is added to the liquid polyol. This can be explained in terms of the molecular weight distribution within the prepolymer. The addition of the liquid polyol to the isocyanate is the preferred method. The lower the viscosity of the prepolymer the lower the resulting density of the formulation.

The polyol is prepared by adding the required quantity of polyol (of the molecular weight required) to a round bottomed flask, placing in the heating mantle and melted. The appropriate volume of water is added and mixed. Appropriate quantities of the remaining components (chain extender, blowing agent, catalysts, surfactant and cross linking agents) are added to the reaction flask and mixed thoroughly for 25 - 30 minutes using a moderate shear rate.

The amount of material required to$\phi$ manufacture even the largest implant or scaffold is only tens of grams. The smallest scaffolds per this invention are only a fraction of a gram. In a preferred embodiment the reaction ingredients are metered using precision gear pumps which can deliver an accuracy of 0.01 g. The materials of the invention are metered into a mixing chamber.

The polyol mixture and isocyanate resin are aggressively mixed in a mixing chamber. The aggressive mixing causes the two incompatible phases to be interspersed. Mixing is crucial to the processes of the invention as it brings the reactive sites to come into close proximity and this facilitates the reactions.

Shot mass per this invention varies from as little as 1 gram. More typically the shot size varies from 2g to 10g. More typically from 3-5g.

Mixing is an important variable in controlling the size and distribution of the voids. The greater the level of material mixing the smaller the diameter of the voids.

In the design of a mixing chamber it is desirable to generate shear and turbulence. This ensures that there is homogeneity locally in the chamber and at different points in the chamber. Shear mixing is measured indirectly by the speed of the mixer or the shear rate or the relative velocities of the components of the mixing unit or the velocity gradient. The degree of turbulence in the chamber is dependent on the design of the mixing chamber and is very difficult to quantify.

The mixing process is characterised in that:

- The mixing chamber is circular in cross-section
- An annular space is provided between the chamber outer wall and an inner relative rotational element.
- The rotational element rotates at speeds in excess of 1000rpm, preferably between 2000rpm and 6000rpm.
- The rotational element has slot features, and/or fin features and/or hole features and/or raised features so as to generate shear stresses and turbulence in the mixing chamber.

The dispensing step is characterised in that the material is ejected out of the mixing chamber and into a mould. The conditions of the mould facilitate a phenomenon known as " free rise". Free rise describes a process whereby the mixed products are permitted to expand without limitation. The mixed reactants are dispensed into a mould that facilitates at least a nine fold volumetric expansion and as much as a 50 fold expansion of the biomaterial. The mould is vented to allow for free rise and is placed preferably in a carbon dioxide rich oven, which assists in the stabilisation of the materials physical structure. The temperature of the mould should be in excess of the temperature of the reactants. Preferably the mould temperature is between 80°C and 90°C. The mould should be manufactured from a material, which is non-reactive to isocyanate. PTFE, silicone polypropylene and POM are exemplary mould materials.

The low density polyurethane biomaterial is placed in a carbon dioxide oven after dispensing into the mould for a minimum of 1 hour. The oven temperature is between 80°C - 90°C but preferably at 80°C. This process is important in that it helps prevent shrinkage of the material and allows virtually all reactive sites within the structure to react.

Reticulation of the scaffold can be carried out by crushing. This optional step increases the number of pores per void. It has the disadvantage that reticulated pores will tend to be more irregular in shape.

It should be obvious to one skilled in the art that decreasing the density of the material causes an increase in the surface area. When the material is in the shape of a low density foam, or cellular material the surface area becomes especially large.

The materials of this invention are two phase materials. The hard phase is generated by the reaction of MDI with small chain molecules and through trimerisation. Typically, the small chain molecules are diols, diamines, alkanol amines or water. Preferably the chain extenders are diols or water. The second phase of the material is referred to as the soft phase and is composed PTMEG or a polycarbonate polyol. The two phases differ in cohesive energy density.

The soft phase typically has a cohesive energy density in the region of 20-22 $MPa^{\frac{1}{2}}$ while the hard phase typically has a cohesive energy density in the region of 24 -28$MPa^{\frac{1}{2}}$. This allows the two phases separate into distinct domains. This phase separated domain structure confers optimal biocompatibility characteristics to the material.

Phase separation is enhanced by achieving most of the crosslinking in the hard phase. The use of a difunctional polyol is preferred.

Preferably the hard segment content is from 35 % to 65%. More preferably the hard segment content is from 35% to 55%. More ideally the hard segment content is from 40 to 50%. The Hard Segment Content is defined as follows:

$$\{M_{(isocyanate)} + M_{(chain\ extender)}) + M_{(crosslinker)} + M_{(water)} - M_{(CO2)} \} /$$

$$\{M_{(isocyanate)} + M_{(chain\ extender)} + M_{(crosslinker)} + M_{(water)} + M_{(polyol)} - M_{(CO2)} \}$$

Where M is the mass of a material used in or generated by the reaction.

The incorporation of isocyanurate linkages in the hard phase contributes strongly to stability of the hard phase. These cyclic structures are highly stable. The incorporation of isocyanurates into a soft tissue contacting material is a unique feature of the invention.

The materials of this invention can be manufactured to indentation hardness which match the compliance of tissues in given applications. The materials of example 1 and 2 have characteristics which approximately match those of tissue. An important advantage of these highly porous foams in tissue applications is that the indentation force does not increase rapidly with the level of indentation. Harder grades of material are easily prepared using already established principles.

## Examples

The unique physical and biostability features of the polyether polyurethane urea isocyanurate and polycarbonate polyurethane urea isocyanurate biomaterials of this invention will be demonstrated by the following examples.

## Example 1

For preparation of porous biostable polytetramethylene oxide polyurethane urea isocyanurate biomaterial, a polyol resin and an isocyanate pre-polymer were prepared. In the preparation of the polyol resin the following raw materials were added to a heated round bottom flask and mixed at 50 - 60°C for a minimum of 25 -30 minutes.

| Raw material | Quantity (php) |
|---|---|
| Terathane (MW 1000)[1] | 100 |
| Triethanolamine[2] | 4.6 |
| Water [2] | 2.56 |
| 1,4 Butanediol [2] | 8.05 |
| BF 2270[3] | 1.0 |
| RC Catalyst 105[4] | 2.96 |
| Desmorapid PP[5] | 0.34 |
| Kac/Deg[6] | 0.73 |

[1]PTMEG (Du Pont),
[2]Sigma Aldrich,
[3]Th GoldSchmidt,
[4]Triethylenediamine (Rhein Chemie),
[5]Whitchem,
[6]Potassium acetate (Sigma Aldrich)

An isocyanate pre-polymer with an NCO content of 15.6% was prepared by charging flake MDI (Desmodur 44M flakes from Bayer, MDI-2,4' isomer content of 1.37%) into a heated round bottom flask and allowing the flakes to melt. Upon complete melting of the MDI, the PTMEG (Terathane MW 1000) was added and the reaction mixture was stirred at 70 - 80°C for 60 to 90 minutes. Dry nitrogen was purged through the reaction flask at all times to eliminate moisture from the reaction vessel.

The polyether polyurethane biomaterial was prepared using metering, mixing and dispensing equipment manufactured by 2KM (Germany). The processing temperature of the materials was 40°C and mixing was achieved at a speed of 5000 RPM. The materials were dispensed into a pre heated silicone mould of temperature 80 - 90°C at an isocyanate index of 1.13. The combined shot size of the materials was 2.2g. The mould was placed in a $CO_2$ vented oven at a temperature of 90°C for a minimum time period of 90 minutes. The biomaterial was reticulated using a hand press.

Samples were taken post curing to perform the tests listed below.

## Results.

The calculated hard segment content of this material was 42.6 %

A sample of the foam was cut using a cutting blade into a rectangular block, ensuring no skin was present on the material. The dimensions were recorded using a calliper and the weight was recorded on an electronic balance. The void content was calculated as 90% based on a solids density of 1200 Kg/m$^3$.

The indentation hardness of the above material was evaluated per ISO standard 2439 with the following deviations

- indenter diameter of 10mm,
- sample thickness of 10mm,

- sample diameter of 20mm.

The sample was prepared by first cutting the sample with a cutting blade and then lasering the appropriate cylinder from the piece of material.

The indentation hardness recorded were 1.14N at 25%, 1.3N at 40% and 4.14N for 65%.

**Biostability**

Samples of this biomaterial were placed in sealed acrylic chambers and implanted in the subcutaneous cavities of rats. The acrylic chambers were sealed with 0.45µm and 3.0µm filters to allow fluids have contact with the biomaterial. The samples were explanted at a number of time points up to 6 months post implantation. The biomaterial samples were examined by Scanning Electron Microscopy (SEM). The images provided in Figures 1.1, 1.3, 1.4 and 1.5 demonstrate that the structural integrity of the biomaterial is comparable to the biomaterial sample that was not subjected to the in vivo environment (Figure 1.2). Histological analysis of this biomaterial (Figure 2.1) demonstrates the presence of a massive and persistent macrophage response. However, despite the presence of a large number of macrophage no evidence of degradation was observed in any of the explanted samples, as demonstrated.

This biomaterial was also implanted in the gluteal muscle of rats and left for up to 6 months. The histological analysis conducted on the explants indicated that;

- ♦ The biomaterial was covered with tissue within 4 weeks of implantation.
- ♦ Angiogenesis was clearly observed within the bulk of the biomaterial 4 weeks following implantation. This anchors the implant in relation to the surrounding tissue.
- ♦ No evidence of biomaterial degradation was observed.

The biomaterial scaffold was implanted in the vasculature of a rabbit for a period of up to 3 months. Again no degradation of the material was observed.

**Example 2**

For preparation of porous biostable polytetramethylene oxide polyurethane urea isocyanurate biomaterial, a polyol resin and an isocyanate pre-polymer were prepared. The polyol is prepared per example 1 using the following chemicals

| Raw material | Quantity (php) |
|---|---|
| Terathane (MW 1000)[1] | 100 |
| Triethanolamine[2] | 4.6 |
| Water [2] | 3.0 |
| 1,4 Butanediol [2] | 8.0 |
| BF 2270[3] | 1.2 |
| RC Catalyst 105[4] | 0.52 |
| DABCO BL11[5] | 0.13 |
| Kac/Deg[6] | 0.73 |

[1]PTMEG (Du Pont),
[2]Sigma Aldrich,
[3]Th GoldSchmidt,
[4]Triethylenediamine solution (Rhein Chemie),
[5]Air Products,
[6] Potassium acetate solution (Sigma Aldrich)

An isocyanate pre-polymer with a NCO content of 25.0% was prepared as per example 1 using PTMEG polyol (Terathane MW 650).

The polytetramethylene oxide polyurethane urea isocyanurate biomaterial was prepared per the method of example 1 at an isocyanate index of 1.13 with the exception that the combined shot size of the components was 1.3g

**Results.**

The calculated hard segment content of this material was 51.9 %

A rectangular block was cut as per example 1 and weighed. The void content was calculated as 95% again based on a base line density of 1200Kg/m$^3$.

**Example 3**

For preparation of a porous biostable polycarbonate polyurethane urea isocyanurate biomaterial, a polyol resin and an isocyanate pre-polymer were prepared.

The polyol is prepared per example 1 using the following chemicals.

| Raw material | Quantity (php) |
|---|---|
| Polycarbonate CX 5510 (MW 1000)[1] | 100 |
| Triethanolamine[2] | 3.6 |
| Water [2] | 3.0 |
| BF 2270[3] | 1.2 |
| RC Catalyst 105[4] | 1.66 |
| DABCO BL 11[5] | 0.8 |
| Kac/Deg[6] | 0.73 |

[1]Nissei Chemical Company, Japan.
[2]Sigma Aldrich
[3]Th GoldSchmidt
[4]Triethylene diamine solution (Rhein Chemie)
[5]Air Products
[6]Potassium acetate solution (Sigma Aldrich)

The isocyanate pre-polymer was prepared as per example 1 using polycarbonate CX 5510 (MW 1000) to produce a prepolymer with an isocyanate content of 15.6%

Polycarbonate CX5510 is a random co-polymer comprising hexamethylene carbonate and pentamethylene carbonate sequences of 1000MW.

The materials were mixed at an isocyanate index of 1.13 in an open top reaction vessel. The temperature of the materials was 40°C and a mixing speed of 2200RPM was achieved. The combined shot size of the materials was 52g.

**Results.**

A rectangular block was cut as per example 1 and weighed. The void content was calculated as 88% based on a solids density of 1200Kg/m$^3$.

Indentation hardness values were measured using the same procedure as per example 1. The following results were obtained: 2.3N at 25%, 3.2N at 40% and 7.8N for 65%.

The invention is not limited to the embodiments hereinbefore described which may be varied in detail.

**Claims**

1. A biostable porous polyurethane foam implant material, the material containing isocyanurate linkages derived from diphenyl methane diisocyanate (MDI), and having a cross-linked structure, and a void content of at least 85%.

2. An implant as claimed in claim 1 wherein the material has a void content in excess of 90%.

3. An implant as claimed in claim 1 wherein the material has a void content in excess of 95%.

4. An implant as claimed in claim 1 wherein the material has a void content in excess of 97%.

5. An implant as claimed in any preceding claim wherein the material is manufactured from

diphenyl methane diisocyanate (MDI) with a 2,4 MDI isomer content of less than 3%;

a linear, long chain diol which is free of tertiary carbon linkages;

water;

a cross-linking agent;

a trimerisation catalyst;

a blowing and/or gelling catalyst; and

a surfactant.

6. An implant as claimed in claim 5 wherein the diol is polytetramethylene ether glycol (PTMEG).

7. An implant as claimed in claim 5 wherein the diol is a polycarbonate diol.

8. An implant as claimed in claim 7 wherein the polycarbonate diol is a reaction product of one or more diols with a carbonate monomer.

9. An implant as claimed in any of claims 5 to 8 wherein the diol molecular weight is between 400 and 5000.

10. An implant as claimed in claim 9 wherein the diol molecular weight is between 500 and 2500.

11. An implant as claimed in any of claims 5 to 10 wherein the trimerisation catalyst is a carboxylate.

12. An implant as claimed in claim 11 wherein the trimerisation catalyst is a potassium acetate.

13. An implant as claimed in claim 12 wherein potassium acetate is present in the reaction formulation in an amount of from 0.02% to 0.12% by mass of the formulation.

14. An implant as claimed in any of claims 5 to 13 wherein the cross-linking agent is present in the reaction formulation in an amount of from 1% to 5% by mass.

15. An implant as claimed in claim 14 wherein the cross-linking agent is trialkanol amine.

16. An implant as claimed in claim 15 wherein the cross-linking agent is triethanolamine.

17. An implant as claimed in any preceding claim wherein the isocyanate index of the reaction formulation is from 1.03 to 1.20.

18. An implant as claimed in claim 17 wherein the index is approximately 1.13.

19. An implant as claimed in any of claims 5 to 18 wherein the reaction formulation includes a chain extender.

20. An implant as claimed in claim 19 wherein the chain extender is a linear aliphatic diol.

21. An implant as claimed in claim 20 wherein the linear aliphatic diol is 1, 4 butane diol.

22. An implant as claimed in any of claims 19 to 21 wherein the chain extender is present in the formulation in an amount of less than 7% by mass.

23. An implant as claimed in claim 22 wherein the chain extender is present in the formulation in an amount of less than 4% by mass.

24. An implant as claimed in any of claims 5 to 23 wherein water is present in the reaction formulation in an amount of from 0.6% to 1.8% by mass.

25. An implant as claimed in any preceding claim wherein the implant has a hard segment content of from 35 to 65%.

26. An implant as claimed in claim 25 wherein the implant has a hard segment content of from 35 to 55%.

27. An implant as claimed in claim 25 or 26 wherein the implant has a hard segment content of from 40 to 50%.

28. An implant as claimed in any preceding claim in the form of an occluder.

29. An implant as claimed in any of claims 1 to 27 in the form of a tissue bridge.

30. A process for preparing a biostable polyurethane implant as claimed in any of claims 1 to 29 comprising the steps of:-

preparing an isocyanate terminated prepolymer in an excess of MDI with a 4, 4 MDI isomer content of greater than 97%;

preparing a diol reaction mixture comprising a diol, a cross-linking agent, a trimerising catalyst, water, a blowing and/or gelling catalyst, and a surfactant;

mixing the prepolymer and the diol;

dispensing the mixed reaction ingredients into a preheated mould which permits free rise of the foam;

post curing the foam;

demoulding the foam; and

machining the foam to form an implant.

31. A process as claimed in claim 30 wherein the foam is post cured in a carbon dioxide rich environment.

32. A process as claimed in claim 30 or 31 including the step of preheating the mould prior to dispensing of the mixed reaction ingredients.

33. A process as claimed in any of claims 30 to 32 including the step, after demoulding, of crushing the foam to increase the open cell content of the foam.

34. A process as claimed in any of claims 30 to 33 wherein the prepolymer is prepared from a prepolymer reaction mixture at a temperature of from 70 to 80°C.

35. A process as claimed in any of claims 30 to 34 wherein the prepolymer reaction mixture is reacted for a period of from 1 to 2 hours.

36. A process as claimed in any of claims 30 to 35 wherein the prepolymer reaction mixture is stirred continuously under a dry inert atmosphere.

37. A process as claimed in any of claims 30 to 36 wherein during moulding the mould temperature is maintained at a temperature of greater than 50°C.

38. A process as claimed in claim 37 wherein the mould temperature is from 80 to 90°C.

39. A process as claimed in any of claims 30 to 38 wherein the post curing is carried out at a temperature of at least 50°C for a period of at least 30 minutes.

40. A process as claimed in claim 39 wherein the post-curing is carried out at a temperature of approximately 80°C.

**41.** A process as claimed in any of claims 30 to 40 wherein the shot size is less than 10g.

**42.** A process as claimed in claim 41 wherein the shot size is less than 5g.

**43.** A process as claimed in claim 41 or 42 wherein the shot size is less than 3g.


**Patentansprüche**

**1.** Implantatmaterial aus biostabilem porösem Polyurethanschaum, wobei das Material Isocyanuratbindungen enthält, die sich von Diphenylmethandiisocyanat (MDI) herleiten, und eine vernetzte Struktur und einen Porengehalt von mindestens 85 % aufweist.

**2.** Implantat nach Anspruch 1, worin das Material einen Porengehalt von über 90 % aufweist.

**3.** Implantat nach Anspruch 1, worin das Material einen Porengehalt von über 95 % aufweist.

**4.** Implantat nach Anspruch 1, worin das Material einen Porengehalt von über 97 % aufweist.

**5.** Implantat nach einem der vorangehenden Ansprüche, worin das Material hergestellt wird aus:

Diphenylmethandiisocyanat (MDI) mit einem 2,4-MDI-Isomer-Gehalt von weniger als 3 %;

einem linearen, langkettigen Diol, das frei von tertiären Kohlenstoffbindungen ist;

Wasser;

einem Vernetzungsmittel;

einem Trimerisationskatalysator;

einem Treib- und/oder einem Gelierkatalysator; und

einem oberflächenaktiven Mittel.

**6.** Implantat nach Anspruch 5, worin das Diol Polytetramethylen-etherglycol (PTMEG) ist.

**7.** Implantat nach Anspruch 5, worin das Diol ein Polycarbonatdiol ist.

**8.** Implantat nach Anspruch 7, worin das Polycarbonatdiol ein Reaktionsprodukt aus einem oder mehreren Diolen mit einem Carbonat-Monomer ist.

**9.** Implantat nach einem der Ansprüche 5 bis 8, worin das Molekulargewicht des Diols zwischen 400 und 5000 liegt.

**10.** Implantat nach Anspruch 9, worin das Molekulargewicht des Diols zwischen 500 und 2500 liegt.

**11.** Implantat nach einem der Ansprüche 5 bis 10, worin der Trimerisationskatalysator Carboxylat ist.

**12.** Implantat nach Anspruch 11, worin der Trimerisationskatalysator ein Kaliumacetat ist.

**13.** Implantat nach Anspruch 12, worin Kaliumacetat in der Reaktionsformulierung in einer Menge von 0,02 Masse% bis 0,12 Masse% der Formulierung vorliegt.

**14.** Implantat nach einem der Ansprüche 5 bis 13, worin das Vernetzungsmittel in der Reaktionsformulierung in einer Menge von 1 Masse% bis 5 Masse% vorliegt.

**15.** Implantat nach Anspruch 14, worin das Vernetzungsmittel Trialkanolamin ist.

**16.** Implantat nach Anspruch 15, worin das Vernetzungsmittel Triethanolamin ist.

**17.** Implantat nach einem der vorangehenden Ansprüche, worin der Isocyanat-Index der Reaktionsformulierung von 1,03 bis 1,20 beträgt.

**18.** Implantat nach Anspruch 17, worin der Index ca. 1,13 beträgt.

**19.** Implantat nach einem der Ansprüche 5 bis 18, worin die Reaktionsformulierung einen Kettenverlängerer einschließt.

**20.** Implantat nach Anspruch 19, worin der Kettenverlängerer ein lineares aliphatisches Diol ist.

**21.** Implantat nach Anspruch 20, worin das lineare aliphatische Diol 1,4-Butandiol ist.

**22.** Implantat nach einem der Ansprüche 19 bis 21, worin der Kettenverlängerer in der Formulierung in einer Menge von weniger als 7 Masse% vorliegt.

**23.** Implantat nach Anspruch 22, worin der Kettenverlängerer in der Formulierung in einer Menge von weniger als 4 Masse% vorliegt.

**24.** Implantat nach einem der Ansprüche 5 bis 23, worin Wasser in der Reaktionsformulierung in einer Menge von 0,6 Masse% bis 1,8 Masse% vorliegt.

**25.** Implantat nach einem der vorangehenden Ansprüche, worin das Implantat einen Hartsegmentgehalt von 35 bis 65 % aufweist.

**26.** Implantat nach Anspruch 25, worin das Implantat einen Hartsegmentgehalt von 35 bis 55 % aufweist.

**27.** Implantat nach Anspruch 25 oder 26, worin das Implantat einen Hartsegmentgehalt von 40 bis 50 % aufweist.

**28.** Implantat nach einem der vorangehenden Ansprüche in der Form eines Okklusionsmittels.

**29.** Implantat nach einem der Ansprüche 1 bis 27 in der Form einer Gewebebrücke.

**30.** Verfahren zur Herstellung eines Implantats aus biostabilem Polyurethan nach einem der Ansprüche 1 bis 29, umfassend die Schritte von:

Herstellen eines Isocyanat-terminierten Vorpolymers in einem Überschuss von MDI mit einem 4,4-MDI-Isomergehalt von größer als 97 %;

Herstellen eines Diolreaktionsgemisches, umfassend ein Diol, ein Vernetzungsmittel, einen Trimerisationskatalysator, Wasser, einen Treib- und/oder Gelierkatalysator, und ein oberflächenaktives Mittel;

Mischen des Vorpolymers und des Diols;

Abgabe der gemischten Reaktionsbestandteile in eine vorgewärmte Form, die freies Ansteigen des Schaums erlaubt;

Nachhärten des Schaums;

Entformen des Schaums; und

maschinelle Verarbeitung des Schaums zum Formen eines Implantats.

**31.** Verfahren nach Anspruch 30, worin der Schaum in einer kohlendioxidreichen Umgebung nachgehärtet wird.

**32.** Verfahren nach Anspruch 30 oder 31, einschließlich des Schrittes des Vorwärmens der Form vor Abgabe der gemischten Reaktionsbestandteile.

33. Verfahren nach einem der Ansprüche 30 bis 32, einschließlich des Schrittes, nach dem Entformen, des Zerkleinerms des Schaums zur Erhöhung des offenzelligen Gehaltes des Schaums.

34. Verfahren nach einem der Ansprüche 30 bis 33, worin das Vorpolymer aus einem Vorpolymerreaktionsgemisch bei einer Temperatur von 70 bis 80°C hergestellt wird.

35. Verfahren nach einem der Ansprüche 30 bis 34, worin das Vorpolymerreaktionsgemisch für eine Zeitdauer von 1 bis 2 Stunden zur Reaktion gebracht wird.

36. Verfahren nach einem der Ansprüche 30 bis 35, worin das Vorpolymerreaktionsgemisch unter einer trockenen inerten Atmosphäre kontinuierlich gerührt wird.

37. Verfahren nach einem der Ansprüche 30 bis 36, worin während des Formens die Formtemperatur bei einer Temperatur von höher als 50°C aufrechterhalten wird.

38. Verfahren nach Anspruch 37, worin die Formtemperatur von 80 bis 90°C beträgt.

39. Verfahren nach einem der Ansprüche 30 bis 38, worin das Nachhärten für eine Zeitdauer von mindestens 30 Minuten bei einer Temperatur von mindestens 50°C durchgeführt wird.

40. Verfahren nach Anspruch 39, worin das Nachhärten bei einer Temperatur von ca. 80°C durchgeführt wird.

41. Verfahren nach einem der Ansprüche 30 bis 40, worin die Schussgröße weniger als 10g beträgt.

42. Verfahren nach Anspruch 41, worin die Schussgröße weniger als 5g beträgt.

43. Verfahren nach Anspruch 41 oder 42, worin die Schussgröße weniger als 3g beträgt.


**Revendications**

1. Matériau d'implant en mousse de polyuréthane poreuse et biostable, le matériau contenant des liaisons isocyanurate dérivées de diphénylméthane diisocyanate (MDI) et ayant une structure réticulée, et une teneur en vide d'au moins 85%.

2. Implant selon la revendication 1, dans lequel le matériau a une teneur en vide supérieure à 90%.

3. Implant selon la revendication 1, dans lequel le matériau a une teneur en vide supérieure à 95%.

4. Implant selon la revendication 1, dans lequel le matériau a une teneur en vide supérieure à 97%.

5. Implant selon l'une quelconque des revendications précédentes, dans lequel le matériau est fabriqué à partir

   de diphénylméthane diisocyanate (MDI) ayant une teneur en isomère MDI 2,4 inférieure à 3% ;

   d'un diol à chaîne longue et linéaire, dépourvu de liaisons de carbone tertiaire ;

   d'eau ;

   d'un agent de réticulation ;

   d'un catalyseur de trimérisation ;

   d'un catalyseur de soufflage et/ou de gélification ; et

   d'un agent tensioactif.

6. Implant selon la revendication 5, dans lequel le diol est le polytétraméthylèneéther glycol (PTMEG).

**7.** Implant selon la revendication 5, dans lequel le diol est un diol de polycarbonate.

**8.** Implant selon la revendication 7, dans lequel le diol de polycarbonate est un produit de réaction d'un ou plusieurs diols avec un monomère de carbonate.

**9.** Implant selon l'une quelconque des revendications 5 à 8, dans lequel le poids moléculaire du diol est compris entre 400 et 5 000.

**10.** Implant selon la revendications 9, dans lequel le poids moléculaire du diol est compris entre 500 et 2 500.

**11.** Implant selon l'une quelconque des revendications 5 à 10, dans lequel le catalyseur de trimérisation est un carboxylate.

**12.** Implant selon la revendication 11, dans lequel le catalyseur de trimérisation est un acétate de potassium.

**13.** Implant selon la revendication 12, dans lequel l'acétate de potassium est présent dans la formulation réactionnelle en une quantité allant de 0,02% à 0,12% en masse de la formulation.

**14.** Implant selon l'une quelconque des revendications 5 à 13, dans lequel l'agent de réticulation est présent dans la formulation réactionnelle en une quantité allant de 1% à 5% en masse.

**15.** Implant selon la revendication 14, dans lequel l'agent de réticulation est une trialcanolamine.

**16.** Implant selon la revendication 15, dans lequel l'agent de réticulation est la triéthanolamine.

**17.** Implant selon l'une quelconque des revendications précédentes, dans lequel l'indice d'isocyanate de la formulation réactionnelle va de 1,03 à 1,20.

**18.** Implant selon la revendication 17, dans lequel l'indice est d'environ 1,13.

**19.** Implant selon l'une quelconque des revendications 5 à 18, dans lequel la formulation réactionnelle comporte un allongeur de chaîne.

**20.** Implant selon la revendication 19, dans lequel l'allongeur de chaîne est un diol aliphatique linéaire.

**21.** Implant selon la revendication 20, dans lequel le diol aliphatique linéaire est le 1,4-butanediol.

**22.** Implant selon l'une quelconque des revendications 19 à 21, dans lequel l'allongeur de chaîne est présent dans la formulation en une quantité inférieure à 7% en masse.

**23.** Implant selon la revendication 22, dans lequel l'allongeur de chaîne est présent dans la formulation en une quantité inférieure à 4% en masse.

**24.** Implant selon l'une quelconque des revendications 5 à 23, dans lequel de l'eau est présente dans la formulation réactionnelle en une quantité allant de 0,6% à 1,8% en masse.

**25.** Implant selon l'une quelconque des revendications précédentes, dans lequel l'implant a une teneur en segment dur allant de 35 à 65%.

**26.** Implant selon la revendication 25, dans lequel l'implant a une teneur en segment dur allant de 35 à 55%.

**27.** Implant selon la revendication 25 ou 26, dans lequel l'implant a une teneur en segment dur allant de 40 à 50%.

**28.** Implant selon l'une quelconque des revendications précédentes, sous forme d'un cache-oeil.

**29.** Implant selon l'une quelconque des revendications 1 à 27, sous forme d'un pont tissulaire.

**30.** Procédé de préparation d'un implant en polyuréthane biostable selon l'une quelconque des revendications 1 à 29, comprenant les étapes :

de préparation d'un prépolymère à terminaison isocyanate dans un excès de MDI ayant une teneur en isomère MDI 4,4 supérieure à 97% ;

de préparation d'un mélange réactionnel de diol comprenant un diol, un agent de réticulation, un catalyseur de trimérisation, de l'eau, un catalyseur de soufflage et/ou de gélification et un agent tensioactif ;

de mélange du prépolymère et du diol ;

de distribution des ingrédients réactionnels mélangés dans un moule préchauffé permettant la montée libre de la mousse ;

de post-cuisson de la mousse ;

de démoulage de la mousse ; et

d'usinage de la mousse pour former un implant.

31. Procédé selon la revendication 30, dans lequel la mousse subit une post-cuisson dans un environnement riche en dioxyde de carbone.

32. Procédé selon la revendication 30 ou 31, comportant l'étape de préchauffage du moule préalablement à la distribution des ingrédients réactionnels mélangés.

33. Procédé selon l'une quelconque des revendications 30 à 32, comportant l'étape, après démoulage, de broyage de la mousse pour augmenter la teneur en cellules ouvertes de la mousse.

34. Procédé selon l'une quelconque des revendications 30 à 33, dans lequel le prépolymère est préparé à partir d'un mélange réactionnel de prépolymère à une température allant de 70 à 80°C.

35. Procédé selon l'une quelconque des revendications 30 à 34, dans lequel le mélange réactionnel de prépolymère est réagi pendant une période allant de 1 à 2 heures.

36. Procédé selon l'une quelconque des revendications 30 à 35, dans lequel le mélange réactionnel de prépolymère est agité en continu sous une atmosphère inerte sèche.

37. Procédé selon l'une quelconque des revendications 30 à 36, dans lequel lors du moulage, la température du moule est maintenue à une température supérieure à 50°C.

38. Procédé selon la revendication 37, dans lequel la température du moule va de 80 à 90°C.

39. Procédé selon l'une quelconque des revendications 30 à 38, dans lequel la post-cuisson est réalisée à une température d'au moins 50°C pendant une période d'au moins 30 minutes.

40. Procédé selon la revendication 39, dans lequel la post-cuisson est réalisée à une température d'environ 80°C.

41. Procédé selon l'une quelconque des revendications 30 à 40, dans lequel la charge d'injection est inférieure à 10 g.

42. Procédé selon la revendication 41, dans lequel la charge d'injection est inférieure à 5 g.

43. Procédé selon la revendication 41 ou 42, dans lequel la charge d'injection est inférieure à 3 g.

008419  25KV    X122      246um

Fig 1.1

Fig 1.2

Fig 1.3

Fig 1.4

Fig 1.5

Fig. 2.1